(19) Europäisches Patentamt
European Patent Office
Office européen des brevets

(11) Veröffentlichungsnummer: **0 046 918**
**B1**

(12) # EUROPÄISCHE PATENTSCHRIFT

(45) Veröffentlichungstag der Patentschrift: **21.09.83**

(51) Int. Cl.³: **C 07 C 39/08,** C 07 C 37/74

(21) Anmeldenummer: **81106368.4**

(22) Anmeldetag: **17.08.81**

(54) **Verfahren zur Herstellung von Brenzcatechin und Hydrochinon.**

(30) Priorität: **30.08.80 DE 3032743**

(43) Veröffentlichungstag der Anmeldung: **10.03.82 Patentblatt 82/10**

(45) Bekanntmachung des Hinweises auf die Patenterteilung: **21.09.83 Patentblatt 83/38**

(84) Benannte Vertragsstaaten: **AT BE CH DE FR GB IT LI NL**

(56) Entgegenhaltungen:
**EP-A-0 022 543**

**INDUSTRIAL & ENGINEERING CHEMISTRY, PRODUCT RESEARCH AND DEVELOPMENT, Band 15, Nr. 3, 1976 J. VARAGNAT "Hydroquinone and Pyrocatechol Production by Direct Oxidation of Phenol" Seiten 212 bis 215**

(73) Patentinhaber: **BAYER AG, Zentralbereich Patente, Marken und Lizenzen, D-5090 Leverkusen 1, Bayerwerk (DE)**

(72) Erfinder: **Jupe, Christoph, Dr., Franz-Peter-Kürten-Weg 2, D-5000 Koeln 80 (DE)**
Erfinder: **Waldmann, Helmut, Dr., Henry-T.-von-Böttinger-Strasse 15, D-5090 Leverkusen (DE)**
Erfinder: **Baumert, Jürgen, An den Kreutzmorgen 7, D-5000 Koeln 80 (DE)**
Erfinder: **Schümmer, Günther, Weissdornweg 1, D-5024 Stommeln (DE)**

**EP 0 046 918 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Verfahren zur Herstellung von Brenzcatechin und Hydrochinon

Die vorliegende Erfindung betrifft ein Verfahren zur Herstellung von Brenzcatechin und Hydrochinon.

Brenzcatechin und Hydrochinon sind technisch wichtige organische Feinchemikalien, die sowohl direkt, z.B. in photographischen Entwicklern, als auch als Zwischenprodukte für z.B. Farbstoffe, Polymerisationsinhibitoren, Pharmazeutika und Pflanzenschutzmittel Verwendung finden [siehe Kirk-Othmer, Encyclopedia of Chemical Technology, 2nd Edition, Vol. 11, Seiten 462 bis 492, insbesondere Seiten 469 und 488 (1966)].

Die Suche nach wirtschaftlichen und einfachen Herstellungsverfahren führte u.a. zu einer Reihe von Phenol-Oxidationsverfahren, die Brenzcatechin und Hydrochinon als Koppelprodukte liefern (siehe z.B. DE-OS 26 58 943, 24 10 742, 23 64 181, 26 58 545, 23 32 747, 15 93 968, 26 33 302, 20 64 497, 21 50 657, 21 67 040, 23 41 743, 24 07 398, 15 43 953, 24 04 114, sowie japanische Patentanmeldungen Nr. 54 55530 und 54 66629 und T. Tsuchiya, M. Andoh und J. Imamura, Nipp. Kag. Kaishi 1979, 3, Seite 370 bis 374).

Bei diesen Verfahren wird Phenol mit einem peroxidischen Reagenz umgesetzt, z.B. mit Wasserstoffperoxid oder einer Percarbonsäure, die meist in einem niedriger als Phenol siedenden Lösungsmittel gelöst sind. Ein weiteres Charakteristikum dieser Verfahren besteht darin, dass zur Vermeidung von Überoxidation ein Unterschuss an Oxidationsmittel, bezogen auf die zu hydroxylierende Verbindung, eingesetzt wird. Dies hat zur Folge, dass nach der Reaktion nicht-umgesetztes Phenol im Reaktionsgemisch enthalten ist.

In der bisher erwähnten Literatur wird zur Aufarbeitung der nach der Hydroxylierung vorliegenden Reaktionsgemische meist auf übliche Methoden verwiesen, vor allem auf Destillation, Extraktion und Kristallisation. Genauere Angaben fehlen weitgehend.

Ausführliche Angaben zur Reaktion und Aufarbeitung findet man in zwei Veröffentlichungen, die technische Anlagen zur Herstellung von Brenzcatechin und Hydrochinon aus Phenol und Wasserstoffperoxid beschreiben, und zwar bei Jean Varagnat, Ind. Eng. Chem., Prod. Res. Dev., Vol. 15, No. 3, Seiten 212 bis 215 (1976) und P. Maggioni und F. Minisci, La Chimica et l'Industria, Vol. 59, No. 4, Seiten 239 bis 242 (1977).

In der Veröffentlichung von J. Varagnat wird dargelegt, wie in einer Folge von fünf Rektifikationskolonnen das Reaktionsgemisch destillativ getrennt wird. Phenol und weitere Hilfs- und Begleitstoffe werden in vier Kolonnen als Kopfprodukte gewonnen und wiederverwendet, während Brenzcatechin und Hydrochinon in einer fünften Kolonne getrennt werden und das Hydrochinon anschliessend einer Kristallisation unterworfen wird.

Auch gemäss dem von P. Maggioni und F. Minisci beschriebenen Verfahren wird das Reaktionsgemisch hauptsächlich destillativ aufgearbeitet. Nach einer stufenweisen Verdampfung in drei hintereinander geschalteten getrennten Verdampfern bei jeweils niedrigerem Druck bis hinunter zu 13 mbar erfolgt die restliche Auftrennung des Gemisches in zwei Rektifikationskolonnen. In der ersten Kolonne wird Phenol zur Rückführung und in der zweiten Kolonne werden die Produkte Brenzcatechin und Hydrochinon erhalten.

Betrachtet man den Aufwand, der für die technische Aufarbeitung von Reaktionsgemischen aus Phenol und peroxidischen Reagenzien zur Gewinnung von Brenzcatechin und Hydrochinon getrieben wird, so kann man feststellen, dass bei den zuvor beschriebenen Verfahren vor allem die Abtrennung des nicht-umgesetzten Phenols einen grossen Aufwand erfordert.

Es wurde nun ein Verfahren zur Herstellung von Brenzcatechin und Hydrochinon durch Umsetzung von Phenol mit einem peroxidischen Hydroxylierungsreagenz und Aufarbeitung des nach der Reaktion und gegebenenfalls nach weiterer Behandlung vorliegenden Gemisches, das nicht-umgesetztes Phenol, ein oder mehrere niedriger als Phenol siedende Lösungsmittel, Brenzcatechin, Hydrochinon und gegebenenfalls weitere Bestandteile enthält, unter Verwendung kontinuierlich betriebener Rektifikationsapparate gefunden, das dadurch gekennzeichnet ist, dass man

a) das Gemisch kontinuierlich einer ersten Rektifikationskolonne, die 3 bis 20 Trennstufen im Abtriebsteil und 5 bis 20 Trennstufen im Verstärkungsteil aufweist, zwischen Verstärkungs- und Abtriebsteil zuführt, diese Kolonne bei einem Druck zwischen 0,02 und 5 bar betreibt, 20 bis 95 Gew.-% des Kopfanfalles kondensiert als flüssigen Rücklauf auf die Kolonne zurückführt, ein Kopfprodukt entnimmt, dass praktisch alle leichter als Phenol siedenden Bestandteile des Zulaufgemisches enthält, und ein Sumpfprodukt abzieht, das Phenol und alle höhersiedenden Anteile des Zulauf-Gemisches enthält und

b) das Sumpfprodukt der ersten Rektifikationskolonne kontinuierlich einer zweiten Rektifikationskolonne, die 3 bis 20 Trennstufen im Abtriebsteil und 3 bis 15 Trennstufen im Verstärkungsteil aufweist, zwischen Abtriebs- und Verstärkungsteil zuführt, diese Kolonne bei einem Druck zwischen 0,003 und 5 bar betreibt, 20 bis 95 Gew.-% des am Kopf anfallenden Produktes kondensiert und als flüssigen Rücklauf oben auf die Kolonne zurückführt, als Kopfprodukt ein reines Phenol entnimmt und ein Sumpfprodukt abzieht, das neben Phenol Brenzcatechin, Hydrochinon und gegebenenfalls weitere Begleitstoffe enthält, und aus dem Sumpfprodukt Brenzcatechin und Hydrochinon isoliert.

In die erfindungsgemässe destillative Aufarbeitung einzusetzende Gemische können erhalten werden, indem man Phenol mit einem peroxidischen Hydroxylierungsreagenz in bekannter Weise umsetzt und gegebenenfalls weiter behandelt.

Peroxidische Hydroxylierungsreagenzien sind solche, die eine -O-O-(Sauerstoff-Sauerstoff)-Gruppe enthalten, z.B. Wasserstoffperoxid oder Percarbonsäuren. Solche Reagenzien werden im allgemeinen als verdünnte Lösungen eingesetzt, es sind jedoch auch Umsetzungen bekannt, in denen solche Reagenzen in hochkonzentrierter oder reiner Form eingesetzt werden (s. z.B. DE-OS 2 064 497).

Die Umsetzungen werden im allgemeinen bis zu einem weitgehenden Umsatz des peroxischen Hydroxylierungsreagenzes durchgeführt. Bevorzugt ist ein Umsatz von über 99%, besonders bevorzugt von mehr als 99,7%, so dass das in die erfindungsgemässe destillative Aufarbeitung einzusetzende Gemisch weitgehend peroxid-frei ist.

Gegebenenfalls kann das aus der Umsetzung von Phenol mit dem peroxidischen Hydroxylierungsreagenz erhaltene Gemisch vor dem Einsatz in die erfindungsgemässe destillative Aufarbeitung einer weiteren Behandlung unterzogen werden. Diese kann z.B. darin bestehen, dass man im Reaktionsgemisch enthaltene Säuren vor einer weiteren Aufarbeitung neutralisiert (s. z.B. DE-OS 2410742). Eine andere Art der weiteren Behandlung kann z.B. darin bestehen, gewisse Bestandteile, z.B. Säure oder Säuren, aus dem Reaktionsgemisch durch Extraktion ganz oder teilweise abzutrennen. Ein solcher Weg wird z.B. von J. Varagnat, Ind. Eng. Chem. Prod. Res. Dev., Vol. 15. No. 3, 1976, S. 212—215 (s. vor allem S. 214, linke Spalte, letzter Absatz) zur Entfernung von Phosphorsäure und Perchlorsäure beschrieben.

Die in die erfindungsgemässe destillative Aufarbeitung einzusetzenden Gemische enthalten Phenol, ein oder mehrere niedriger als Phenol siedende Lösungsmittel, Brenzcatechin, Hydrochinon und gegebenenfalls weitere Bestandteile.

Der Gehalt an Phenol kann in weiten Grenzen schwanken. Im allgemeinen liegt er zwischen 5 und 95 Gew.-%, vorzugsweise zwischen 20 und 90 Gew.-%. Es ist jedoch auch möglich, Gemische mit davon abweichenden Phenol-Gehalten in die erfindungsgemässe destillative Aufarbeitung einzusetzen.

Neben dem Gehalt an Phenol ist zur wirtschaftlichen Durchführung der erfindungsgemässen destillativen Aufarbeitung das Gewichtsverhältnis von Phenol zu Brenzcatechin und Hydrochinon im einzusetzenden Gemisch von Bedeutung.

Im allgemeinen kann das Gewichtsverhältnis von Phenol zu Dihydroxybenzolen beispielsweise zwischen 0,8 und 50 zu 1 liegen. Bevorzugt setzt man Gemische mit einem möglichst geringen Phenolanteil, bezogen auf die Summe von Phenol, Brenzcatechin und Hydrochinon, ein. Die untere Grenze des Phenol-Anteiles ist nicht durch die erfindungsgemässe destillative Aufarbeitung, sondern dadurch bedingt, dass in der der erfindungsgemässen destillativen Aufarbeitung vorgelagerten Hydroxylierungsreaktion ein Phenolüberschuss, bezogen auf das Hydroxylierungsreagenz, erforderlich ist, um eine wirtschaftliche Nutzung sowohl des umgesetzten Phenols als auch des eingesetzten Hydroxylierungsreagenzes zu erreichen (s. z.B. DE-OS 2407398 und DE-OS 2064497).

In der Regel wird man deshalb ein Verhältnis von 10 Gew.-Teilen Phenol zu 1 Gewichtsteil Dihydroxybenzolen im einzusetzenden Gemisch nicht zugunsten eines geringeren Phenol-Anteils unterschreiten. Andererseits ist es für die erfindungsgemässe destillative Aufarbeitung vorteilhaft, ein Verhältnis von 35 Gewichtsteilen Phenol zu 1 Gewichtsteil Dihydroxybenzolen in Richtung auf einen höheren Phenolanteil nicht zu überschreiten, da sonst der Aufwand zur Aufarbeitung grösser wird.

Der Gehalt an niedriger als Phenol siedenden Lösungsmitteln kann ebenfalls in weiten Grenzen schwanken. Im allgemeinen kann er zwischen 2 und 98 Gew.-% liegen oder zwischen 5 und 60 Gew.-%. Bei solchen Lösungsmitteln, die unter den Bedingungen der erfindungsgemässen destillativen Aufarbeitung phenol-haltige azeotrope Gemische bilden, ist ihr Anteil im einzusetzenden Gemisch so klein zu halten, dass daraus nicht das gesamte Phenol azeotrop destilliert werden kann.

Das bedeutet z.B. für den Fall, dass das Phenol/Wasser-Azeotrop bei Normaldruck in der ersten Kolonne als Kopfprodukt erhalten wird, dass die Wassermenge im eingesetzten Gemisch kleiner als das 9,86-fache der Phenolmenge ist, da ein Gewichtsteil Wasser 0,1414 Gewichtsteile Phenol azeotrop mitschleppt. (S. z.B. R.C. Weast, Handbook of Chemistry and Physics, Chemical Rubber Co., Cleveland, Ohio, 1977, 58. Aufl., Seite D 32, System Nr. 620).

Bevorzugt ist das einzusetzende Gemisch so zusammengesetzt, dass keine phenolhaltigen azeotropen Gemische als Kopfprodukte im erfindungsgemässen Verfahren auftreten.

Die niedriger als Phenol siedenden Lösungsmittel können auf verschiedene Art und Weise in das einzusetzende Gemisch gelangt sein. Es kann sich beispielsweise um ein Lösungsmittel oder um mehrere Lösungsmittel handeln.

Solche Lösungsmittel können z.B. als Lösungsmittel für das Hydroxylierungsreagenz verwendet worden sein und zusammen mit diesem in die Umsetzung mit dem Phenol eingesetzt worden sein.

Beispielsweise können gemäss der DE-OS 2364181 Aceton, Essigsäureethylester und Gemische aus Aceton und Essigsäuremethylester als Lösungsmittel für verschiedene Percarbonsäuren verwendet werden.

Niedriger als Phenol siedende Lösungsmittel können aber auch z.B. als Nebenprodukt aus dem Hydroxylierungsreagenz entstanden sein. So liefert beispielsweise die Hydroxylierung mit Wasserstoffperoxid eine äquivalente Menge an Wasser nach folgender Gleichung:

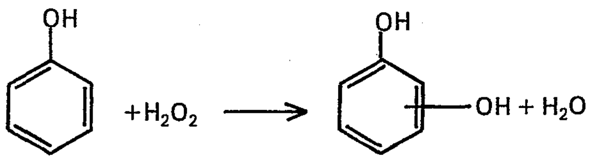

Eine Hydroxylierung mit Peressigsäure liefert z.B. die der abreagierten Peressigsäuremenge äquivalente Menge Essigsäure nach folgender Gleichung:

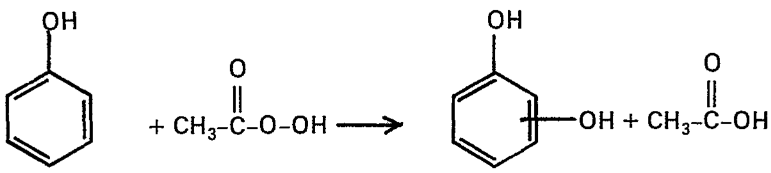

Lösungsmittel, z.B. Kohlenwasserstoffe oder chlorierte Kohlenwasserstoffe, können als Teil des Reaktionsmediums der Hydroxylierungs-Reaktion verwendet werden, so dass beispielsweise auch auf diesem Wege niedriger als Phenol siedende Lösungsmittel in das einzusetzende Gemisch eingebracht worden sein können.

Auch eine zwischen Hydroxylierungsreaktion und erfindungsgemässer destillativer Aufarbeitung gegebenenfalls durchgeführte weitere Behandlung kann zum Vorhandensein von niedriger als Phenol siedenden Lösungsmitteln im einzusetzenden Gemisch führen. So wird z.B. in dem von J. Varagnat beschriebenen Verfahren (s. J. Varagnat Ind. Eng. Chem. Prod. Res. Dev. Vol. 15, No. 3, Seiten 212–215 (1976)) Diisopropyläther dem Reaktionsgemisch der Hydroxylierung zugesetzt, um bei der Extraktion des Gemisches mit Wasser eine gute Phasentrennung zu erzielen.

Bevorzugt setzt man Gemische mit möglichst wenig leichter als Phenol siedenden Lösungsmitteln in die erfindungsgemässe destillative Aufarbeitung ein. Der minimale Gehalt ist im allgemeinen nicht durch die erfindungsgemässe destillative Aufarbeitung, sondern durch vorgeschaltete Verfahrensstufen bedingt. Beispielsweise ist es technisch üblich, Wasserstoffperoxid als 30, 50 oder 70 gew.-%ige wässrige Lösung zu verwenden, da höher konzentrierte wässrige Lösungen von Wasserstoffperoxid ab etwa 85 Gew.-% detonationsfähig sind. Somit befindet sich in einem derartigen Reaktionsgemisch sowohl Wasser, das sich während der Reaktion mit Phenol aus Wasserstoffperoxid gebildet hat, als auch Wasser, das zusammen mit dem Wasserstoffperoxid in die Reaktion eingebracht wurde.

Aus welchen chemischen Substanzen das oder die niedriger als Phenol siedenden Lösungsmittel bestehen, ist für die erfindungsgemässe destillative Aufarbeitung im allgemeinen nicht entscheidend. Der Begriff «Lösungsmittel» ist hier so zu verstehen, dass die damit gekennzeichneten Bestandteile des einzusetzenden Gemisches unter den Bedingungen der erfindungsgemässen destillativen Aufarbeitung bevorzugt nicht oder nur zu einem Teil mit sich selber, untereinander oder mit anderen Bestandteilen des einzusetzenden Gemisches reagieren. Erwünscht ist es, möglichst wenig an niedriger als Phenol siedenden Lösungsmitteln während der erfindungsgemässen destillativen Aufarbeitung durch Reaktionen zu verbrauchen.

Im allgemeinen können durch Reaktionen des oder der Lösungsmittel während der erfindungsgemässen destillativen Aufarbeitung bis zu 80% des Anteils eines oder mehrerer Lösungsmittel des einzusetzenden Gemisches verbraucht werden. Bevorzugt ist es allerdings, höchstens 10%, ganz besonders bevorzugt, unter 5% eines oder mehrerer Lösungsmittel des einzusetzenden Gemisches durch Reaktionen zu verbrauchen.

Als Lösungsmittel, die niedriger als Phenol sieden und im einzusetzenden Gemisch alleine oder im Gemisch vorhanden sein können, seien beispielhaft genannt: Wasser, 1,2-Dichlorpropan, Essigsäure, Essigsäuremethylester, Essigsäureethylester, Aceton, Diisopropylether, Propionsäure, iso-Buttersäure, Benzol, Toluol, o-Xylol, m-Xylol, p-Xylol, Dioxan, 1,2-Dichloräthan, 1,1,2-Trichlorethan.

Die in die erfindungsgemässe destillative Aufarbeitung einzusetzenden Gemische enthalten die Dihydroxybenzole Brenzcatechin und Hydrochinon. Der Gehalt an diesen Substanzen kann in weiten Grenzen schwanken. Dies gilt ebenfalls für das Verhältnis von Brenzcatechin zu Hydrochinon.

Im allgemeinen liegt der Gehalt an Dihydroxybenzolen zwischen 0,1 und 95 Gew.-%. Obwohl ein möglichst grosser Gehalt an Dihydroxybenzolen bevorzugt ist, liegt wegen des nach der Reaktion vorliegenden nicht-umgesetzten Phenols und wegen des oder der leichter als Phenol siedenden Lösungsmittel in vielen Fällen ein Gehalt von unter 60 Gew.-% an Dihydroxybenzolen im einzusetzenden Gemisch vor. Häufig liegt der Gehalt an Dihydroxybenzolen im einzusetzenden Gemisch zwischen 0,5 und 10 Gew.-%.

Das Gewichtsverhältnis von Brenzcatechin zu Hydrochinon ist für das erfindungsgemässe Verfahren nicht von Bedeutung. Im allgemeinen liegt dieses Verhältnis zwischen 0,1 und 10 oder zwischen 0,8 und 4.

Die in die erfindungsgemässe destillative Aufarbeitung einzusetzenden Gemische können neben den bisher aufgeführten und erläuterten Bestandteilen einen oder mehrere weitere Bestandteile enthalten.

Der Gehalt an weiteren Bestandteilen kann in weiten Grenzen schwanken, er kann z.B. zwischen 0,1 und 50 Gew.-% liegen. Ein möglichst geringer Gehalt an weiteren Bestandteilen ist bevorzugt.

Die gegebenenfalls vorhandenen weiteren Bestandteile können Siedepunkte unter oder oberhalb des Siedepunktes von Phenol haben, sie können auch sehr hochsiedend oder nicht-destillierbar sein.

Falls weitere Bestandteile ganz oder teilweise, gegebenenfalls im Gemisch mit anderen, bereits erwähnten Bestandteilen des einzusetzenden Gemisches unter den Bedingungen der erfindungsgemässen destillativen Aufarbeitung phenol-haltige azeotrope Gemische bilden, ist ihr Anteil im einzusetzenden Gemisch so klein zu halten, dass daraus nicht das gesamte Phenol azeotrop abdestilliert werden kann.

Die weiteren Bestandteile haben im allgemeinen die gleichen chemischen Eigenschaften wie die bereits erwähnten niedriger als Phenol siedenden Lösungsmittel. D.h., sie sind unter den Bedingungen der erfindungsgemässen destillativen Aufarbeitung chemisch weitgehend oder zumindest ausreichend inert.

Bevorzugt setzt man in die erfindungsgemässe destillative Aufarbeitung Gemische ein, die, wenn sie weitere Bestandteile im obigen Sinne enthalten, nur solche weiteren Bestandteile enthalten, deren Siedepunkte entweder niedriger als der Siedepunkt des Phenols oder höher als der Siedepunkt des Brenzcatechins liegen. Es sind also solche Gemische bevorzugt, in denen keine zwischen Phenol und Brenzcatechin siedenden Substanzen enthalten sind. Ganz besonders bevorzugt sind Gemische, die, wenn sie weitere Bestandteile im obigen Sinne enthalten, nur solche weiteren Bestandteile enthalten, deren Siedepunkte entweder niedriger als der Siedepunkt des niedrigst-siedenden Lösungsmittels oder des niedrigst-siedenden Lösungsmittelgemisches liegen und/oder höher als der Siedepunkt des Brenzcatechins.

Für die destillative Abtrennung von Brenzcatechin und Hydrochinon ist es darüber hinaus von ganz besonderem Vorteil, nur solche Gemische in die erfindungsgemässe destillative Aufarbeitung einzusetzen, die, so sie weitere Bestandteile im obigen Sinne enthalten, nur solche weiteren Bestandteile enthalten, die entweder niedriger als das niedrigst siedende Lösungsmittel oder Lösungsmittelgemisch sieden und/oder die höher sieden als Hydrochinon und/oder die unter den Bedingungen der erfindungsgemässen destillativen Aufarbeitung nicht destillierbar sind.

Die gegebenenfalls vorhandenen weiteren Bestandteile können auf verschiedenen Wegen in das einzusetzende Gemisch gelangt sein. Sie können chemisch von höchst unterschiedlicher, teilweise nicht einmal genau bestimmbarer Natur sein.

Als Beispiele für weitere Bestandteile seien genannt:

Sauerstoff, der z.B. aus der Zersetzung eines peroxidischen Reagenzes stammen kann;

Kohlenoxide ($CO$, $CO_2$), die z.B. durch Überoxidation in der Hydroxylierungsreaktion entstanden sein können;

Salze von Säuren, die z.B. bei der Neutralisation von Säuren im Reaktionsgemisch entstanden sein können;

Trihydroxybenzole, die z.B. bei der Hydroxylierungsreaktion als Nebenprodukte entstanden sein können;

höher als Hydrochinon siedende oder nicht destillierbare organische Substanzen, die sich z.B. ähnlich wie Braunkohlen oder wie Huminsäuren oder teerartig verhalten können und die z.B. als Nebenprodukte der Hydroxylierungsreaktion entstanden sein können;

höher als Hydrochinon siedende oder nicht-destillierbare Phosphor-enthaltende Substanzen oder andere höher als Hydrochinon siedende Substanzen mit metallkomplexierenden Eigenschaften, die z.B. dem Hydroxylierungsgemisch zugesetzt worden sein können oder die sich aus zugesetzten Komplexbildern gebildet haben können.

Gut geeignet für die erfindungsgemässe destillative Aufarbeitung sind z.B. Gemische, die durch Umsetzung von Phenol mit der Lösung einer Monopercarbonsäure mit 2 bis 4 Kohlenstoffatomen in einem organischen Lösungsmittel mit einem Siedepunkt über 100°C (bei Normaldruck) erhalten wurden. Solche Gemische können als niedriger als Phenol siedende Bestandteile das Lösungsmittel, die der Percarbonsäure entsprechende Carbonsäure und bis zu 5 Gew.-% Wasser enthalten. Insbesondere kommen Gemische in Frage, die als niedriger als Phenol siedende Bestandteile Benzol, Propionsäure und bis zu 5 Gew.-% Wasser enthalten.

Das Einsatzgemisch wird zur Durchführung der erfindungsgemässen destillativen Aufarbeitung kontinuierlich einer ersten Rektifikationskolonne zwischen Verstärkungs- und Abtriebsteil zugeführt.

Diese Kolonne weist 3 bis 20 Trennstufen im Abtriebsteil und 5 bis 20 Trennstufen im Verstärkungsteil auf.

Bevorzugt sind 5 bis 18, ganz besonders bevorzugt 9 bis 16 Trennstufen im Abtriebsteil. Für den Verstärkungsteil sind 5 bis 18 Trennstufen bevorzugt, besonders bevorzugt sind hierfür 7 bis 16 Trennstufen.

Eine Trennstufe ist hier und im folgenden definiert als ein Kolonnenabschnitt, dessen Trennwirkung bei einer Rektifikation einer einmaligen Gleichgewichtseinstellung zwischen aufsteigender Dampf- und absteigender Flüssigkeitsphase gleichkommt, wie es z.B. erläutert ist in «Organikum, Organisch-Chemisches Grundpraktikum», 15. Aufl. (Nachdruck), VEB Deutscher Verlag der

Wissenschaften, Berlin 1977, Seiten 63-69, vor allem Seiten 66 und 67.

Diese Kolonne wird bei einem Druck zwischen 0,02 und 5 bar betrieben. Bevorzugt betreibt man die Kolonne bei einem Druck zwischen 0,1 bis 1,2 bar. Ganz besonders bevorzugt ist ein Betriebsdruck zwischen 0,2 und 1,1 bar.

Die Temperaturen in der Kolonne stellen sich entsprechend dem Druck und der Zusammensetzung der Stoffgemische an den verschiedenen Stellen der Kolonne ein. Man betreibt die gesamte erfindungsgemässe destillative Aufarbeitung vorteilhaft so, dass produktseitig eine Temperatur von 250°C nicht überschrittten wird. Bevorzugt ist es, produktseitige Temperaturen zwischen 230°C und 250°C höchstens kurzzeitig zu erreichen. Ganz besonders bevorzugt ist es, produktseitig stets Temperaturen unterhalb von 230°C vorliegen zu haben.

Die untere Grenze für Druck bzw. Temperatur ist durch den Erstarrungspunkt des Kopfproduktes gegeben. Die durch Druck und Zusammensetzung des Kopfproduktes bedingte Siedetemperatur muss oberhalb des Festpunktes des Kopfproduktes liegen, da sonst eine Rektifikation unter Aufgabe eines flüssigen, aus kondensiertem Kopfprodukt bestehenden Rücklaufes, nicht durchgeführt werden kann.

Ein Teil des am Kopf der Kolonne anfallenden Dampfes wird kondensiert und flüssig als sog. «Rücklauf» auf die Kolonne zurückgegeben. Der nicht als Rücklauf auf die Kolonne zurückgegebene Teil des Kopfproduktes wird entnommen (sog. «Entnahme»). Im allgemeinen liegt das Gewichtsverhältnis von Rücklauf zu Entnahme (R:E) zwischen 0,25 und 19. Bevorzugt ist ein Verhältnis R:E zwischen 0,5 und 10. Ganz besonders bevorzugt ist ein Verhältnis R:E zwischen 0,8 und 5.

Man entnimmt ein Kopfprodukt, das praktisch alle niedriger als Phenol siedenden Bestandteile des eingesetzten Gemisches enthält. D.h., man betreibt die Kolonne mit einer solchen Energiezufuhr, dass im Sumpf nur noch wenig niedriger als Phenol siedende Bestandteile enthalten sind.

Der Gehalt an niedriger als Phenol siedenden Bestandteilen im Sumpf kann zwischen 0,005 und 2 Gew.-% liegen. Bevorzugt ist innerhalb dieses Bereiches ein möglichst niedriger Gehalt an niedriger als Phenol siedenden Bestandteilen.

Der Gehalt an Phenol und höher als Phenol siedenden Komponenten im Kopfprodukt ist gering. Er kann zwischen 0,005 und 5 Gew.-% liegen. Bevorzugt ist ein Phenolgehalt von 0,5 bis 2 Gew.-%.

Das entnommene Kopfprodukt kann einer beliebigen Verwendung zugeführt werden. Wenn das Kopfprodukt mehr als eine Substanz enthält, kann man es z.B. einer weiteren Auftrennung zuführen, um die einzelnen Substanzen als reine Stoffe zu gewinnen.

Bevorzugt ist es, Lösungsmittel rein zu gewinnen und erneut, z.B. als Lösungsmittel für peroxidische Hydroxylierungsreagenzien, zu verwenden. Ebenso ist es vielfach von Vorteil, ein aus einem peroxidischen Hydroxylierungsmittel entstandenes Lösungsmittel, z.B. aus Perisobuttersäure entstandene Isobuttersäure, in reiner Form zu gewinnen und erneut zur Herstellung der entsprechenden peroxidischen Verbindung (in gegegebenem Beispiel: Perisobuttersäure) einzusetzen.

Das aus der ersten Rektifikationskolonne der erfindungsgemässen destillativen Aufarbeitung erhaltene Sumpfprodukt wird kontinuierlich einer zweiten Rektifikationskolonne zwischen Abtriebs- und Verstärkungsteil zugeführt. Die zweite Rektifikationskolonne weist 3 bis 20 Trennstufen im Verstärkungsteil und 3 bis 15 Trennstufen im Abtriebsteil auf.

Bevorzugt ist es, als zweite Rektifikationskolonne eine Kolonne mit 4 bis 10 Trennstufen im Verstärkungsteil und 4 bis 8 Trennstufen im Abtriebsteil einzusetzen, ganz besonders bevorzugt sind 5 bis 7 Trennstufen im Verstärkungsteil und 5 bis 6 Trennstufen im Abtriebsteil.

Die zweite Rektifikationskolonne wird bei einem Druck zwischen 0,003 und 5 bar, bevorzugt zwischen 0,01 und 1,2 bar und ganz besonders bevorzugt zwischen 0,02 und 0,8 bar betrieben.

Das Kopfprodukt ist ein weitgehend reines Phenol mit einem Phenolgehalt von über 95 Gew.-%. Bevorzugt wird ein Kopfprodukt mit über 99 Gew.-%, ganz besonders bevorzugt ein Kopfprodukt mit über 99,8 Gew.-% Phenol erhalten.

Das Kopfprodukt wird teilweise kondensiert und in flüssiger Form als Rücklauf oben auf die Rektifikationskolonne zurückgegeben.

Der Anteil, der als Rücklauf auf die Kolonne zurückgegeben wird, liegt zwischen 20% und 95% des gesamten am Kopf anfallenden Produktes. Bevorzugt ist es 50 bis 75% des am Kopf anfallenden Produktes als Rücklauf zu verwenden. Das entspricht einem Verhältnis von Rücklauf zu Entnahme (R:E) zwischen 1:1 und 3:1. Besonders bevorzugt ist es, 55% bis 65% des am Kopf anfallenden Produktes als Rücklauf zu verwenden.

Der nicht als Rücklauf verwendete Teil des Kopfproduktes wird entnommen und kann beliebig verwendet werden. Im allgemeinen wird man ihn kondensieren und in flüssiger oder fester Form weiterverwenden.

Bevorzugt ist es, das als Kopfprodukt entnommene Phenol, gegebenenfalls nach weiterer Reinigung oder sonstiger Behandlung, erneut in die Umsetzung mit dem Hydroxylierungsreagenz einzusetzen.

Besonders bevorzugt ist es, sowohl das Kopfprodukt der ersten als auch das Kopfprodukt der zweiten Rektifikationskolonne ganz oder teilweise im Verfahren zur Herstellung von Brenzcatechin und Hydrochinon und/oder einer seiner Vorstufen zu verwenden.

Man entnimmt im allgemeinen den grössten Teil der im Zulauf befindlichen Phenol-Menge als Kopfprodukt der zweiten Rektifikationskolonne. Es ist bevorzugt, soviel Phenol am Kopf zu entnehmen, dass im Sumpfprodukt ein Gehalt von 30 bis 70 Gew.-% Phenol vorliegt.

Besonders bevorzugt liegt im Sumpf ein Phenolgehalt von 40 bis 65 Gew.-% vor.

Man zieht ein Sumpfprodukt ab, das Phenol, Brenzcatechin, Hydrochinon und sonstige höher als Phenol siedende Bestandteile enthält.

Aus diesem Sumpfprodukt werden Brenzcatechin und Hydrochinon isoliert. Dies kann in üblicher Weise erfolgen, z.B. durch Destillation, Extraktion, Rektifikation und/oder Kristallisation. Ein besonders geeignetes Verfahren ist z.B. in der deutschen Patentanmeldung P 29 28 553.8 beschrieben.

Für die Durchführung der erfindungsgemässen destillativen Aufarbeitung sind alle üblichen Rektifikationskolonnen geeignet, wie z.B. Füllkörper- oder Bodenkolonnen. Auch Kolonnen mit Gewebe- oder anderen Packungen sind gut geeignet. Ebenso ist die Art der zu den Kolonnen gehörenden Verdampfer für die Durchführung der erfindungsgemässen Aufarbeitung nicht entscheidend, da alle gängigen Verdampfer-Typen eingesetzt werden können, wie z.B. Rohrbündel-Wärmeaustauscher oder Fallfilmverdampfer mit Zwangsumlauf.

Für die Fertigung der Rektifikationsapparaturen können alle technisch üblichen Werkstoffe eingesetzt werden, die im Temperaturbereich bis etwa 250°C gegenüber den zu trennenden Substanzen hinreichend stabil sind. Geeignet sind z.B. Glas, Titan und hochlegierte Edelstähle mit Chrom- und/oder Nickelgehalten von jeweils über 10 Gew.-%, z.B. Werkstoffe gemäss DIN 1.4571 oder DIN 1.4439 oder V4A-Stahl.

Eine vorteilhafte Durchführungsform der erfindungsgemässen destillativen Aufarbeitung ist im folgenden dargestellt:

Das einzusetzende Gemisch wird durch Umsetzung von Phenol mit der Lösung einer Percarbonsäure mit 2 bis 4 Kohlenstoffatomen in einem organischen Lösungsmittel erhalten und enthält:

8–15 Gew.-Teile eines unter 100°C siedenden organischen Lösungsmittels wie Benzol, 1,2-Dichlor-propan oder Essigsäureethylester
0,5–2 Gew.-Teile Wasser
3–8 Gew.-Teile einer Mono-Carbonsäure mit 2 bis 4 Kohlenstoffatomen
50–90 Gew.-Teile Phenol
1,5–4 Gew.-Teile Brenzcatechin
0,6–3 Gew.-Teile Hydrochinon
0,2–2 Gew.-Teile Hochsieder (davon 2 bis 4 Gew.-% anorganische Salze, der Rest organischer Natur mit einem Gewichtsverhältnis Kohlenstoff: Sauerstoff: Wasserstoff von 60:80:15 bis 25:2:8).

Das Gemisch wird einer Füllkörper- oder Siebbodenrektifikationskolonne aus Glas oder Edelstahl V4A, die 4 bis 8 theoretische Trennstufen im Verstärkungs- und 4 bis 8 theoretischen Trennstufen im Abtriebsteil aufweist, zwischen Abtriebs- und Verstärkungsteil zugeführt.

Die Kolonne wird bei einem Kopfdruck von 200 bis 1100 mbar betrieben. Das Verhältnis von Rücklauf zu Entnahme liegt zwischen 0,5:1 und 8:1.

Das Kopfprodukt enthält praktisch die Gesamtmenge an Lösungsmittel, Wasser und Carbonsäure. Der Phenol-Gehalt im Kopfprodukt liegt unter 0,01 Gew.-%. Das Kopfprodukt wird in einer weiteren Rektifikationskolonne in ein Kopfprodukt aus dem organischen Lösungsmittel und Wasser und ein Sumpfprodukt aus der Carbonsäure und einer Spur Phenol getrennt. Beide Produkte werden erneut zur Herstellung der Lösung der Percarbonsäure eingesetzt.

Das Sumpfprodukt, das weniger als 0,1 Gew.-% an niedriger als Phenol siedenden Bestandteilen enthält, wird einer zweiten Rektifikationskolonne zwischen Verstärkungs- und Abtriebsteil zugeführt. Diese zweite Rektifikationskolonne weist 3 bis 5 Trennstufen sowohl im Antriebs- als auch im Verstärkungsteil auf und wird bei einem Kopfdruck von 20 bis 800 mbar betrieben, das Verhältnis von Rücklauf zu Entnahme liegt zwischen 1,1:1 und 4:1.

Die Hauptmenge des im Zulauf zur zweiten Kolonne befindlichen Phenols wird als reines Phenol, das weniger als 0,3 Gew.-% niedriger als Phenol siedende und weniger als 0,01 Gew.-% höher als Phenol siedende Bestandteile enthält, am Kopf entnommen und der Hydroxylierungsreaktion zugeführt.

Das Sumpfprodukt, das etwa 50 Gew.-% Phenol, Brenzcatechin, Hydrochinon und Höhersieder enthält, wird abgezogen und einer weiteren Aufarbeitung zur Gewinnung von Brenzcatechin und Hydrochinon zugeführt, z.B. nach dem in der deutschen Patentanmeldung P 29 28 553.8 beschriebenen Verfahren.

Gegenüber den bekannten Verfahren zur Auftrennung solcher oder ähnlicher Gemische ist der Aufwand verringert. Die erfindungsgemässe destillative Aufarbeitung hat darüber hinaus den Vorteil, dass die im Verfahren auftretenden Hochsieder, die im allgemeinen hochsiedende Oxidationsprodukte des Phenols sind, gelöst bleiben und weder in den Verdampfern, Sümpfen noch anderswo in den Kolonnen ausfallen, so dass Verstopfungen vermieden und Ablagerungen auf ein sehr geringes Mass beschränkt bleiben.

Es ist als überraschend anzusehen, dass mit einem gegenüber bekannten Verfahren einfacheren Verfahren ein Phenol als Kopfprodukt erhalten werden kann, das ohne aufwendige Massnahmen erneut in die Hydroxylierungsreaktion eingesetzt werden kann. Der Fachmann hätte in Kenntnis der bekannten Verfahren vielmehr erwartet, dass eine verbesserte Durchführung des Verfahrens nur mit erhöhtem apparativen und energetischen Aufwand möglich sein würde.

Beispiel:

Das folgende Beispiel soll die Erfindung näher erläutern, ohne sie irgendwie einzuschränken.

1,09 kg/h eines Gemisches, das durch Umsetzung von Phenol mit einer Lösung von Perpropionsäure in einem Gemisch aus Benzol und Propionsäure erhalten wurde, wurde kontinuierlich der Mitte einer Rektifikationskolonne zugeführt.

Im zugeführten Gemisch wurden folgende Komponenten bestimmt

| | |
|---|---|
| Benzol | 11,47 Gew.-% |
| Propionsäure | 6,50 Gew.-% |
| Wasser | 0,25 Gew.-% |
| Phenol | 77,30 Gew.-% |
| Brenzcatechin | 2,48 Gew.-% |
| Hydrochinon | 1,56 Gew.-% |

Der nicht bestimmte Rest von 0,45 Gew.-% bestand aus verschiedenen Substanzen, die höher als Hydrochinon oder nicht unzersetzt siedeten.

Die Rektifikationskolonne war aus Glas und hatte eine wirksame Länge von 3000 mm. Sie war mit Glas-Raschigringen der Masse 4 × 4 mm gefüllt, der innere Kolonnendurchmesser betrug 50 mm.

Vor Eintritt in die Kolonne wurde das Gemisch in einem gläsernen Wärmeaustauscher, der mit Wärmeträgeröl beheizt wurde, auf 90°C aufgeheizt.

Die Kolonne wurde unter einem Druck von 250 mbar (am Kopf der Kolonne gemessen) gehalten.

Am Sumpf befand sich ein ebenfalls aus Glas gefertigter Verdampfer mit konvektivem Umlauf, der mit 200°C heissem Wärmeträger-Öl beheizt wurde. Im Sumpf wurde eine Temperatur von 142–143°C eingestellt, am Kopf 73–82°C gemessen. Pro Stunde fiel eine Menge von 1,4 kg Produkt als Destillat am Kopf der Kolonne an.

Das Kopfprodukt wurde kondensiert, 1,2 kg/h wurden als flüssiger Rücklauf auf den Kopf der Kolonne zurückgegeben, 0,2 kg/h wurden entnommen.

Das Kopfprodukt hatte folgende Zusammensetzung:

| | |
|---|---|
| Benzol | 62,5 Gew.-% |
| Propionsäure | 35,0 Gew.-% |
| Wasser | 1,33 Gew.-% |
| Phenol | 1,17 Gew.-% |
| | 100,00 Gew.-% |

Aus dem entnommenen Kopfprodukt wurden Benzol und Propionsäure isoliert und bei der Herstellung einer Lösung von Perpropionsäure in Benzol/Propionsäure eingesetzt.

Aus dem Sumpf der Kolonne wurden 0,89 kg/h Produkt entnommen und der Mitte einer zweiten Rektifikationskolonne zugeführt.

Im entnommenen Sumpfprodukt wurden folgende Bestandteile analysiert:

| | |
|---|---|
| Propionsäure | 0,09 Gew.-% |
| Phenol | 94,42 Gew.-% |
| Brenzcatechin | 3,03 Gew.-% |
| Hydrochinon | 1,91 Gew.-% |
| | 99,45 Gew.-% |

Die zweite Rektifikationskolonne hatte folgende Masse: wirksame Länge 2400 mm, innerer Durchmesser 50 mm, Füllung: 4 × 4 mm Glasraschigringe; Werkstoff für Kolonne und Verdampfer: Glas.

Vor dem Eintritt in die Kolonne wurde das Gemisch auf 140°C aufgeheizt.

Die Kolonne wurde unter einem Druck von 667 mbar, am Kopf der Kolonne gemessen, betrieben. Die Temperaturen betrugen im Sumpf 181 bis 182°C und im Kopf 166 bis 167°C.

Die Kolonne war mit einem Verdampfer mit konvektivem Umlauf versehen, der mit Wärmeträgeröl von 230°C beheizt wurde.

Am Kopf der Kolonne fiel eine Menge von 1,89 kg/h Produkt an, die kondensiert wurde.

Vom Kopfanfall wurden 1,13 kg/h als Rücklauf flüssig auf die Kolonne zurückgegeben.

Im Kopfprodukt wurden analysiert:

| | |
|---|---|
| Propionsäure | 0,08 Gew.-% |
| Phenol | 99,92 Gew.-% |
| Brenzcatechin Spur | |
| Hydrochinon Spur | (jeweils unter 0,01 Gew.-%) |

Das Kopfprodukt wurde zur Umsetzung mit Perpropionsäure eingesetzt.

Aus dem Sumpf der zweiten Rektifikationskolonne wurden pro Stunde 0,13 kg Produkt entnommen, in dem folgende Bestandteile analytisch nachgewiesen wurden.

| | |
|---|---|
| Phenol | 62,23 Gew.-% |
| Brenzcatechin | 20,62 Gew.-% |
| Hydrochinon | 13,00 Gew.-% |

Aus dem Sumpfprodukt wurden durch weitere Rektifikationen Phenol, Brenzcatechin und Hydrochinon isoliert. Das Phenol wurde zur Umsetzung mit Perpropionsäure eingesetzt.

**Patentansprüche**

1. Verfahren zur Herstellung von Brenzcatechin und Hydrochinon durch Umsetzung von Phenol mit einem peroxidischen Hydroxylierungsreagenz und Aufarbeitung des nach der Reaktion und gegebenenfalls nach weiterer Behandlung vorliegenden Gemisches, das nichtumgesetztes Phenol, ein oder mehrere niedriger als Phenol siedende Lösungsmittel, Brenzcatechin, Hydrochinon und gegebenenfalls weitere Bestandteile enthält, unter Verwendung weiterer Bestandteile enthält, unter Verwendung kontinuierlich betriebener Rektifikationsapparate, dadurch gekennzeichnet, dass man

a) das Gemisch kontinuierlich einer ersten Rektifikationskolonne, die 3 bis 20 Trennstufen im Abtriebsteil und 5 bis 20 Trennstufen im Verstärkungsteil aufweist, zwischen Verstärkungs- und Abtriebsteil zuführt, diese Kolonne bei einem Druck zwischen 0,02 und 5 bar betreibt, 20 bis 95 Gew.-% des Kopfanfalles kondensiert als flüssigen Rücklauf auf die Kolonne zurückführt, ein Kopfprodukt entnimmt, das praktisch alle leichter als Phenol siedende Bestandteile des Zulaufgemisches enthält, und ein Sumpfprodukt abzieht, das Phenol und alle höhersiedenden Anteile des Zulauf-Gemisches enthält und

b) das Sumpfprodukt der ersten Rektifikationskolonne kontinuierlich einer zweiten Rektifikationskolonne, die 3 bis 20 Trennstufen im Abtriebsteil und 3 bis 15 Trennstufen im Verstärkungsteil aufweist, zwischen Abtrieb- und Verstärkungsteil zuführt, diese Kolonne bei einem Druck zwischen 0,003 und 5 bar betreibt, 20 bis 95 Gew.-% des am Kopf anfallenden Produktes kondensiert und als flüssigen Rücklauf oben auf die Kolonne zurückführt, als Kopfprodukt ein reines Phenol entnimmt und ein Sumpfprodukt abzieht, das neben Phenol Brenzcatechin, Hydrochinon und gegebenenfalls weitere Begleitstoffe enthält, und aus dem Sumpfprodukt Brenzcatechin und Hydrochinon isoliert.

2. Verfahren nach Anspruch 1, dadurch gekennzeichnet, dass das einzusetzende Gemisch folgende Anteile enthält:

| | |
|---|---|
| Unter 100°C siedendes organisches Lösungsmittel | 8 bis 15 Gew.-Teile |
| Wasser | 0,5 bis 2 Gew.-Teile |
| Monocarbonsäure mit 2 bis 4 C-Atomen | 3 bis 8 Gew.-Teile |
| Phenol | 50 bis 90 Gew.-Teile |
| Brenzcatechin | 1,5 bis 4 Gew.-Teile |
| Hydrochinon | 0,6 bis 3 Gew.-Teile |
| Hochsieder | 0,2 bis 2 Gew.-Teile |

3. Verfahren nach Anspruch 1, dadurch gekennzeichnet, dass im einzusetzenden Gemisch als niedriger als Phenol siedende Lösungsmittel ein organisches Lösungsmittel mit einem Siedepunkt unter 100°C (bei Normaldruck), eine Monocarbonsäure mit 2 bis 4 C-Atomen und bis zu 5 Gew.-% Wasser enthalten sind.

4. Verfahren nach Anspruch 3, dadurch gekennzeichnet, dass das Gemisch Benzol, Propionsäure und bis zu 5 Gew.-% Wasser enthält.

5. Verfahren nach Ansprüchen 1 bis 4, dadurch gekennzeichnet, dass man produktseitig Temperaturen von 230°C nicht überschreitet.

6. Verfahren nach Ansprüchen 1 bis 5, dadurch gekennzeichnet, dass man die erste Rektifikationskolonne bei einem Kopfdruck zwischen 0,1 und 1,2 bar und die zweite Rektifikationskolonne bei einem Kopfdruck zwischen 0,02 und 0,8 bar betreibt.

7. Verfahren nach Ansprüchen 1 bis 6, dadurch gekennzeichnet, dass man als Kopfprodukt der zweiten Rektifikationskolonne ein Produkt erhält, das über 99 Gew.-% Phenol enthält.

8. Verfahren nach Ansprüchen 1 bis 7, dadurch gekennzeichnet, dass man die erste Rektifikationskolonne bei einem Rücklaufverhältnis R:E (Rücklauf: Entnahme) zwischen 0,5:1 und 10:1 (Gewichtsteilen) und die zweite Rektifikationskolonne bei einem Rücklaufverhältnis R:E zwischen 1:1 und 3:1 betreibt.

9. Verfahren nach Ansprüchen 1 bis 8, dadurch gekennzeichnet, dass die erste Rektifikationskolonne

| | |
|---|---|
| Im Verstärkerteil | 5 bis 18 Trennstufen |
| und im Abtriebsteil | 5 bis 18 Trennstufen |

und die zweite Rektifikationskolonne

| | |
|---|---|
| im Verstärkerteil | 4 bis 10 Trennstufen |
| und im Abtriebsteil | 4 bis 8 Trennstufen |

aufweist.

10. Verfahren nach Ansprüchen 1 bis 9, dadurch gekennzeichnet, dass man die Kopfprodukte der ersten und zweiten Rektifikationskolonne ganz oder teilweise erneut im Verfahren zur Herstellung von Brenzcatechin und Hydrochinon und/oder in einer seiner Vorstufen verwendet.

**Claims**

1. Process for the preparation of pyrocatechin and hydroquinone by reacting phenol with a peroxidic hydroxylating reagent and working up the mixture which is present after the reaction, if appropriate after further treatment, this mixture containing unreacted phenol, one or more solvents which boil at a temperature lower than phenol, pyrocatechin, hydroquinone and, if appropriate, further constituents, using rectifying apparatuses which are operated continuously, characterised in that

a) the mixture is fed continuously, between the concentrating and the refining part, to a first rectification column which has 3 to 20 separation stages in the refining part and 5 to 20 separation stages in the concentrating part, this column is operated at a pressure between 0,02 and 5 bar, 20 to 95% by weight of the top product is condensed and fed back to the column as liquid reflux, a top product which contains virtually all the constituents of the feed mixture which boil more easily than phenol is extracted, and a bottom product is drawn off which contains phenol and all fractions of the feed mixture which boil at a higher temperature and

b) the bottom product of the first rectification column is fed continuously, between the refining and the concentrating part, to a second rectification column which has 3 to 20 separation stages in the refining part and 3 to 15 separation stages in the concentrating part, this column is operated at a pressure between 0.003 and 5 bar, 20 to 95% by weight of the product obtained at the top is condensed and returned to the top of the column as liquid reflux,pure phenol is extraceted as the top product and a bottom product is drawn off which, in addition to phenol, contains pyrocatechin, hydroquinone and, if appropriate, further accompanying substances, and pyrocatechin and hydroquinone are isolated from the bottom product.

2. Process according to Claim 1, characterised in that the mixture to be used contains the following fractions:

| | |
|---|---|
| organic solvent boiling below 100°C | 8 to 15 parts by weight |
| water | 0.5 to 2 parts by weight |
| monocarboxylic acid having 2 to 4 c atoms | 3 to 8 parts by weight |

| | |
|---|---|
| phenol | 50 to 90 parts by weight |
| pyrocatechin | 1.5 to 4 parts by weight |
| hydroquinone | 0,6 to 3 parts by weight |
| high boiling products | 0.2 to 2 parts by weight |

3. Process according to Claim 1, characterised in that the mixture to be used contains, as the solvent which boils at a lower temperature than phenol, an organic solvent having a boiling point lower than 100°C (under atmospheric pressure), a monocarboxylic acid having 2 to 4 C atoms and up to 5% by weight of water.

4. Process according to Claim 3, characterised in that the mixture contains benzene, propionic acid and up to 5% weight of water.

5. Process according to Claims 1 to 4, characterised in that the products do not exceed temperatures of 230°C.

6. Process according to Claims 1 to 5, characterised in that the first rectification column is operated at a head pressure between 0.1 and 1.2 bar and the second rectification column is operated at a head pressure between 0.02 and 0.8 bar.

7. Process according to Claims 1 to 6, characterised in that the top product of the second rectification column is one which contains more than 99% by weight of phenol.

8. Proces according to Claims 1 to 7, characterised in that the first rectification column is operated at a reflux ratio R:E (reflux: extraction) between 0.5:1 and 10:1 (parts by weight) and the second rectification column is operated at a reflux ratio R:E between 1:1 and 3:1.

9. Process according to Claims 1 to 8, characterised in that the first rectification column has

| | |
|---|---|
| in the | |
| concentrating part | 5 to 18 separation stages |
| and in the refining part | 5 to 18 separation stages |

and the second rectification column has

| | |
|---|---|
| in the | |
| concentrating part | 4 to 10 separation stages |
| and in the refining part | 4 to 8 separation stages. |

10. Process according to Claims 1 to 9, characterised in that the top products of the first and second rectification columns are used again, completely or partially, in the process for the preparation of pyrocatechin and hydroquinone and/or in one of its preliminary stages.

**Revendications**

1. Procédé de production de pyrocatéchol et d'hydroquinone par réaction de phénol avec un réactif peroxydique d'hydroxylation et traitement du mélange se présentant après la réaction et, les cas échéant, après un autre traitement, qui contient du phénol n'ayant pas réagi, un ou plusieurs autres solvants de point d'ébullition plus bas que celui du phénol, du pyrocatéchol, de l'hydroquinone et, le cas échéant, d'autres composants, en utilisant des appareils de rectification à marche continue, caractérisé en ce que

a) on fait arriver le mélange en continu entre la partie concentration et la partie élimination d'une première colonne de rectification qui présente 3 à 20 étages de séparation dans la partie élimination et 5 à 20 étages de séparation dans la partie concentration, on fait fonctionner cette colonne à une pression comprise entre 0,02 et 5 bars, on recycle à la colonne 20 à 95% en poids de la fraction de tête condensée comme reflux liquide, on prélève un produit de tête qui contient pratiquement tous les composants du mélange introduit de plus bas point d'ébullition que le phénol et on soutire un produit de queue qui contient le phénol et tous les composants de plus haut point d'ébullition du mélange introduit et

b) on fait arriver en continu le produit de queue de la première colonne de rectification entre la partie élimination et la partie concentration d'une seconde colonne de rectification qui présente 3 à 20 étages de séparation dans la partie élimination et 3 à 15 étages de séparation dans la partie concentration, on fait fonctionner cette colonne à une pression comprise entre 0,003 et 5 bars, on condense 20 à 95% en poids du produit obtenu en tête et on le recycle à la partie supérieure de la colonne comme reflux liquide, on prélève comme produit de tête un phénol pur et on soutire un produit de queue qui contient, à côté de phénol, du pyrocatéchol, de l'hydroquinone et, le cas échéant, d'autres impuretés, et on isole du produit de queue le pyrocatéchol et l'hydroquinone.

2. Procédé suivant la revendication 1, caractérisé en ce que le mélange à utiliser contient les composants suivants:

| | |
|---|---|
| solvant organique bouillant | |
| au-dessous de 100°C | 8 à 15 parties en poids |
| eau | 0,5 à 2 parties en poids |
| acide monocarboxylique | |
| de 2 à 4 atomes | |
| de carbone | 3 à 8 parties en poids |
| phénol | 50 à 90 parties en poids |
| pyrocatéchol | 1,5 à 4 parties en poids |
| hydroquinone | 0,6 à 3 parties en poids |
| composants | |
| de haut point d'ébullition | 0,2 à 2 parties en poids. |

3. Procédé suivant la revendication 1, caractérisé en ce que le mélange à utiliser contient comme solvants de plus bas point d'ébullition que le phénol, un solvant organique ayant un point d'ébullition inférieur à 100°C (à la pression normale), un acide monocarboxylique de 2 à 4 atomes de carbone et jusqu'à 5% en poids d'eau.

4. Procédé suivant la revendication 3, caractérisé en ce que le mélange contient du benzène, de l'acide propionique et jusqu'à 5% en poids d'eau.

5, Procédé suivant les revendications 1 à 4, caractérisé en ce que des températures de 230°C ne sont pas dépassées du côté du produit.

6. Procédé suivant les revendications 1 à 5, caractérisé en ce qu'on fait fonctionner la première

colonne de rectification à une pression de tête comprise entre 0,1 et 1,2 bar et la seconde colonne de rectification à une pression de tête comprise entre 0,02 et 0,8 bar.

7. Procédé suivant les revendications 1 à 6, caractérisé en ce qu'on obtient comme produit de tête de la seconde colonne de rectification un produit qui contient plus de 99% en poids de phénol.

8. Procédé suivant les revendications 1 à 7, caractérisé en ce qu'on fait fonctionner la première colonne de rectification à un rapport de reflux R:P (reflux: prélèvement) compris entre 0,5:1 et 10:1 (parties en poids) et on fait fonctionner la seconde colonne de rectification à un rapport de reflux R:P compris entre 1:1 et 3:1.

9. Procédé suivant les revendications 1 à 8, caractérisé en ce que la première colonne de rectification présente

| | |
|---|---|
| dans la partie concentration | 5 à 18 étages de séparation |
| et dans la partie élimination | 5 à 18 étages de séparation |

et la seconde colonne de rectification présente

| | |
|---|---|
| dans la partie concentration | 4 à 10 étages de séparation |
| et dans la partie élimination | 4 à 8 étages de séparation. |

10. Procédé suivant les revendications 1 à 9, caractérisé en ce qu'on réutilise les produits de tête de la première et de la seconde colonne de rectification totalement ou partiellement dans le procédé de production de pyrocatéchol et d'hydroquinone et/ou dans l'une de ses étapes préliminaires.